**Europäisches Patentamt**

**European Patent Office**

**Office européen des brevets**

(19)

(11) Publication number : **0 371 049 B1**

## EUROPEAN PATENT SPECIFICATION

(12)

(45) Date of publication of patent specification :
03.06.92 Bulletin 92/23

(51) Int. Cl.⁵ : **G01N 33/569**, G01N 33/571, G01N 33/543

(21) Application number : **88905974.7**

(22) Date of filing : **12.07.88**

(86) International application number :
PCT/GB88/00561

(87) International publication number :
WO 89/00695 26.01.89 Gazette 89/03

(54) **METHOD FOR THE DIAGNOSIS OF INFECTIONS WITH DETECTION OF LIPOPOLYSACCHARIDE ANTIGENS.**

(30) Priority : **14.07.87 GB 8716489**
**28.05.88 GB 8812802**

(43) Date of publication of application :
06.06.90 Bulletin 90/23

(45) Publication of the grant of the patent :
03.06.92 Bulletin 92/23

(84) Designated Contracting States :
**AT BE CH DE FR GB IT LI LU NL SE**

(56) References cited :
EP-A- 193 431
EP-A- 194 789
EP-A- 264 036
WO-A-83/03678
WO-A-85/02685
US-A- 4 497 899
JOURNAL OF IMMUNOLOGY, vol.138, no.2, 15
January 1987; Y.X.ZHANG et al., pp.575-581

(56) References cited :
INFECTION & IMMUNITY, vol.44, no.2, May
1984; H.D.CALDWELL et al., pp.306-314
JOURNAL OF CLINICAL MICROBIOLOGY,
vol.22, no.4, October 1985; R.BARNES et al.,
pp.609-613
ANALYTICAL BIOCHEMISTRY, vol.137, 1984;
W.BRADBURY et al., pp.129-133
JOURNAL OF IMMUNOLOGICAL METHODS,
vol.76, 1985; W.BRADBURY et al., pp.299-305

(73) Proprietor : **THE VICTORIA UNIVERSITY OF MANCHESTER**
Oxford Road
Manchester. M13 9PL (GB)

(72) Inventor : **RICHMOND, Shirley, Jean**
The Firs Fallowfield
Manchester M14 6HE (GB)
Inventor : **STOREY, Christopher, Charles**
31 Talbot Road Fallowfield
Manchester M14 6GA (GB)

(74) Representative : **Tunnicliffe, Peter Barry**
Westfields House 5 Vincent Drive Lache Lane
Chester, CH4 7RQ (GB)

## Description

This invention relates to a diagnostic method useful in biological screening procedures, more particularly in methods for detecting the presence of infectious agents, and especially for the diagnosis of bacterial infections such as those of chlamydial infections, for example those of Chlamydia trachomatis.

A known technique for the diagnosis of infectious agents which cause disease is to employ a procedure by which some antigenic component of the infectious agent (usually an infectious organism) is immunologically recognised. This has the advantage that it does not require the use of culture procedures (which can be slow) or the use of viable organisms in the laboratory, so that the agents or organisms do not have to be maintained in a viable state until they reach a laboratory, and enables results to be obtained more speedily.

The most recent procedures are described as Enzyme Linked Immuno-Sorbent Assays ("ELISA" tests), and kits for carrying them out are available commercially. In these tests, the antigen derived from the infectious agent is first captured from a fluid sample by means of a plastic carrier which has an affinity for the antigen and then the presence of the captured antigen on the carrier is detected by treatment with an antibody which reacts with it. The detecting antibody is usually "tagged" with an ancillary agent which can subsequently be used to reveal its presence and position on the carrier; in ELISA tests the antibody is conjugated with an enzyme which, when contacted with an appropriate substrate or reagent, generates a colour which indicates the presence of the enzyme and, therefore, also of the antigen to which the antibody binds.

Conventionally, the plastic carrier does not itself have any affinity for the desired antigen, and has to be made receptive to it by first being coated with a layer of material which incorporates an antibody to which the desired antigen attaches. Consequently, the system is relatively complicated because of the involvement of so many components.

ELISA tests are usually carried out in "microtitre wells" in plastic plates or strips, which make them very suitable for the bulk testing of large numbers of samples. Like all new rapid diagnostic tests, it is important that they are both sensitive (so that they do not miss the truly positive samples) and also specific (so that they do not produce false positive reactions) if they are to provide an efficient and satisfactory alternative to older conventional diagnostic methods.

We have now found that these problems can be overcome for the diagnosis of infectious agents, for example bacteria, with a distinct lipopolysaccharide antigen, notably those of the genus Chlamydia, by utilising the ability of a contacting absorptive medium (especially nitrocellulose) which acts as an efficient medium for capturing lipopolysaccharide antigens.

Indeed, such a medium has proved to be more efficient for this purpose than a plastic base coated with a "capture" antibody, as is used in a conventional ELISA test.

Nitrocellulose membranes are used regularly as absorbents for nucleic acids. They are also used for protein separation procedures, in which the proteins are first separated from each other in a polyacrylamide gel and then transferred from the polyacrylamide gel to a nitrocellulose membrane, placed in contact with the gel, by application of an electrical potential across the two - i.e. by electrophoretic action.

Hitherto, however, it has not been realised that a nitrocellulose membrane is sensitive enough to detect the low concentration of antigen present in clinical material. It is particularly surprising that it can absorb a lipopolysaccharide antigenic component derived from an infectious organism as opposed to a proteinaceous antigenic component.

Thus according to the present invention we provide an improved diagnostic method for the diagnosis of infection by an infectious agent with a distinct lipopolysaccharide antigen, in which the sample under examination is first intimately applied to a contacting absorptive medium which has an affinity for a lipopolysaccharide, which then absorbs the lipopolysaccharide antigen of the infectious agent, and the resulting support carrying the absorbed antigen then is treated with a solution containing an antibody which (a) has an affinity for the said antigen and also (b) is associated with a means whereby the presence of the antibody (and consequently of any antigen to which it is attached) can be rendered detectable, wherein the clinical sample, before it is applied to the contacting absorptive medium, is treated with a proteolytic enzyme.

The term "contacting absorptive medium" is used here as meaning that the medium exerts its affinity for the antigen, without being restricted to any specific mode or mechanism by which it does so.

The invention is especially applicable to infectious agents with a distinct epitope on a lipopolysaccharide antigen, and organisms for which it is especially useful include those of the genus Chlamydia.

The method of the present invention is useful for the biological screening of large numbers of samples with ease and speed. Taking the case of the chlamydial organisms as an example, it also has the advantage that, as it relies upon the detection of the heat-stable lipopolysaccharide antigen, which is genus-specific. Thus, for example, it can be used to diagnose chlamydial infections other than those of Chlamydia trachomatis alone, which affects humans. Especially it can also be used to diagnose infections of Chlamydia psittaci, which affect animals and birds, and so can be valuable for diagnostic work in the veterinary field. This is in contrast to the methods dependent upon the protein antigens, which are less heat-stable and are not genus-specific but specific only to an individual species, for example Chlamydia trachomatis.

Another advantage of the method is that samples for test can be transported and stored under much less stringent conditions than those which usually need to be used when the viability of an organism has to be maintained, and mild cooling (for example a few degrees below 0 degrees C.) may be used instead of deep-freezing.

The sample for examination should be in the form of a dispersion and/or solution of the sample material in an aqueous medium and may be prepared by extracting the initial clinical sample material with water or an aqueous buffer solution.

This aqueous mixture containing the sample material is then preferably heated to assist in extracting the desired soluble lipopolysaccharide antigen, to kill any viable infectious agents or organisms present and destroy infectivity, and also to assist in insolubilising or deactivating other components of the sample which could interfere with the accuracy of the diagnosis. The time and temperature of heating may vary, but conveniently at least 10 minutes at about the boiling point (100 degrees C.) may be used.

In our experience, we have found that false positive reactions can occur when there is protein A in the clinical specimen. This problem can arise, in the case of Chlamydia, when other organisms are present in addition to Chlamydia, notably in the case of eye swabs when there is Staphylococcus aureus infection of the conjunctiva. Since Chlamydia trachomatis sometimes infects the eyes of babies and young adults, it is important to have a Chlamydia diagnostic test can be used satisfactorily on conjunctival swabs.

We have now found that an enzyme treatment of the specimen can be used to destroy proteinaceous materials (for example protein A) present, and even to destroy it completely. The antigen detected in the diagnostic method of the present invention is a lipopolysaccharide, which resists treatment with proteolyticenzymes, and we have found that treatment of the clinical specimens with such an enzyme (protease) before it is applied to the contacting absorptive medium improves both the sensitivity and the specificity of the test. Moreover, this treatment not only improves the ability of the test to detect the lipopolysaccharide antigen, but also enables specimens to contact or pass through the contacting absorptive medium more easily, so that it is less likely that the specimen will clog the contacting absorptive medium and centrifigation of the specimen as part of its preparation can be made no longer necessary.

This modification has very significantly improved our test.

Thus according to a further feature of our invention we also provide an improvement in the said diagnostic method which comprises treatment of the clinical sample, before it is applied to the contacting absorptive medium, with a proteolytic enzyme.

The proteolytic enzyme used may be any of those known in the art, and may be used under conventional conditions, for example temperature, media and times, which are sufficient to enable it to act to attack and break down the proteinaceous materials present.

We also prefer that, after the enzyme treatment, the specimen should be treated in order to deactivate the enzyme as this might otherwise adhere to the contacting absorptive medium and destroy a labelled (e.g. radio-labelled) monoclonal antibody used at the later stage of the test procedure. This deactivating treatment may be a heating treatment, especially at 100 degrees C or higher; conveniently, this may be achieved by boiling or, still more conveniently, by steam treatment in a pressure vessel.

The intimate contacting of the sample under examination with the contacting absorptive medium may be achieved by using the contacting absorptive medium in a form of high surface area. Most preferably this is in the form of a thin film, conveniently termed a membrane, but other forms of high surface area may be used.

The liquid sample may be contacted with the contacting absorptive medium in any way which allows adequate intimacy of contact and sufficient time for the lipopolysaccharide antigen to be taken up by the contacting absorptive medium. This can be done by allowing the liquid sample to stand in contact with the contacting absorptive medium but the preferred method is for the liquid sample to be impelled through a layer, and especially a film (membrane), of the contacting absorptive medium. This can be done by application of a pressure differential between the sides of the layer or film, so that the liquid is either blown or sucked through it.

The contacting absorptive medium may be any material known in the art to have an affinity for materials or products of a lipopolysaccharide nature. The preferred contacting absorptive medium is nitrocellulose, as this has exceptional efficiency and convenience in use.

The nitrocellulose (which may alternatively be described as cellulose nitrate) should preferably be in a pure form, as additives can interfere.

The contacting absorptive medium, especially when in the form of a film or membrane (for example of nitrocellulose) should be sufficiently permeable to allow the liquid of the sample to pass through it at a convenient speed. This can be achieved very conveniently by using a film (for example of pure nitrocellulose) having pore sizes of the order of about half a micron, but materials having larger or smaller pore sizes may be used if desired.

The contacting absorptive medium carrying the absorbed antigen is preferably washed thoroughly (conveniently with water) before being treated with a solution of an antibody in the next stage.

The antibody then used to treat antigen-bearing contacting absorptive medium may be any which has an affinity for the lipopolysaccharide antigen, but is preferably a monoclonal antibody for increased specificity and sensitivity.

The means, associated with the said antibody, whereby the presence of the antibody (and consequently of any antigen to which it is attached) can be rendered detectable may be any means which results in the antibody being capable of being detected, directly or indirectly. The preferred means, however, is for the antibody to be radio-labelled (i.e. rendered radioactive) so that its presence can be detected either directly by the radiation emitted or indirectly by the effect of the emitted radiation on a photographic emulsion or other photosensitive material. The radio-labelling can be carried out with any radio-isotope which can be incorporated in the antibody without adversely affecting its antibody properties (i.e. its affinity for the antigen), and preferably has a half-life which allows for convenient and safe handling int the laboratory. An example of this labelling by treating the antibody with radioactive ioding (Isotope $^{125}$I). The technique for radio-labelling with radioactive iodine can be carried out for example by the technique described by Hunter and Greenwood (Nature, 1962, Volume 194, page 495). This radiolabelling provides a highly sensitive label and requires only very small amounts of the antibody.

The contacting absorptive medium (for example a film of nitrocellulose), after it has absorbed the lipopolysaccharide antigen from the sample, should then be then treated with a material which blocks all the remaining attachment sites and prevents it trapping any further antigens or materials which can obscure the absorbed lipopolysaccharide antigen. The most convenient reagent for this purpose is a solution of high protein content, which may be buffered appropriately if desired.

The invention is especially well suited for use in an immune blot technique (or dot immunobinding technique) in which impermeable plates or sheets of glass or plastic, provided with a series of holes, are used to carry out tests on a number of samples simultaneously. For this, two such plates are clamped together with the series of holes in each substantially in register with each other, and with a sheet of the contacting absorptive medium (for example nitrocellulose film) clamped between them. The samples to be assessed are then put into the wells formed by the holes in one plate and the liquid is drawn through the film of contacting absorptive medium by applying suction below the second plate. The lipopolysaccharide antigen is trapped in the film layer and then, when the plates are removed from it, the film can be treated with a solution of the antibody bearing its labelling material. The trapped antigen is thus labelled in its position on the film, and the film can be developed or used to provide a visible or measurable record of the range of samples.

When the labelling is by way of a radio-labelled antibody, it is very convenient to prepare the "record" by placing the contacting absorptive medium (for example nitrocellulose film), bearing its labelled "spots" of trapped antigen with the attendant radio-labelled antibody, against a sheet of radiation-sensitive material -- for example X-Ray film -- and then developing the exposed material in the customary fashion.

This has the advantage of producing a record of many samples in a form which lends itself very well to being kept as a permanent record and examined quantitatively as well as qualitatively. Also, if the amount of radioactivity present is small and the initial exposure to the radiation-sensitive film is too dark or too faint to be studied adequately, the procedure can be repeated again using fresh film and a different exposure, and this may be repeated as often as is desired so long as the radioactivity is still present.

It is particularly useful, however, as it is very sensitive and can enable an observer to distinguish very readily those samples which show a strong reaction.

For practical purposes there is also the advantage that, in addition to increased sensitivity, the immune blot test is sampler to perform than the ELISA test as fewer washing steps are involved. Once each specimen has been placed in its individual well, the whole film of contacting absorptive medium, for example nitrocellulose membrane, (which can, for example, test about 90 specimens) is processed and the washing and subsequent treatment of individual wells, which is necessary in ELISA tests, is avoided.

The invention is not restricted to this specific form of multi-sample test, but may if desired be applied to tests using fewer samples -- even individual samples in individual wells -- if desired.

The invention is illustrated but not limited by the following Examples, in which the parts and percentages are by weight unless stated otherwise.

EXAMPLE 1:

Preparation of clinical samle for test:

A swab taken of a mucous surface to be assessed is treated with an aqueous phosphate buffer solution containing sucrose, as transit medium, and this is then heated for 15 to 30 minutes at 100 degrees C. in a boiling water bath. This helps to solubilise the material for test, and also destroys any infectivity. The liquid is then centrifuged lightly, to remove any insoluble materials which could cause clogging of the membrane in the subsequent stages.

Test procedure:

The resulting liquid is then put into a "well" of a plate as used for dot immunobinding techniques. Such plates are well known and are available commercially under the name "Bio-dot" (Bio-Rad Laboratories Ltd., Watford WDI.8A, United Kingdom), and comprise a sheet of plastic or glass in which a multiplicity of holes have been made (commonly about 90 holes).

Two such plates are clamped together with a thin sheet of nitrocellulose membrane (pure nitrocellulose, available as BA85 grade, and with pore size of approximately 0.45 micron) between them, so that the holes in the two plates correspond and form a series of cavities ("wells") each with the nitrocellulose membrane across it.

The quantity of liquid per well is usually about 400 microlitres, and liquid derived by the above procedure from a variety of samples may be put into the different wells. Control samples of known content (usually at least one which is inert and one which contains a known amount of the antigen sought) are put into some of the wells to act as standard. Then the liquid in the various wells is drawn through the nitrocellulose membrane by suction from below the plates.

The nitrocellulose membrane is then separated from the plates and soaked for about 1 hour in a phosphate-buffered saline solution containing 5% of of powdered skimmed milk. This high protein solution serves to block all the remaining attachment sites on the membrane. The membrane is then washed very thoroughly with water to remove all surplus protein.

The washed membrane is then treated with a very dilute aqueous solution of a monoclonal antibody which has an affinity for the lipopolysaccharide antigen and also has been radio-labelled with radioactive iodine ($^{125}$I) by the method of Hunter and Greenwood (Nature, 1962, Volume 194, page 495), by soaking in the solution for about 2 hours at ambient temperature.

The membrane is then removed from the antibody solution and washed very thoroughly, especially to remove all radioactive material which is not attached to the antigen on the nitrocellulose membrane, and dried.

The dried membrane is then put into an intensifying screen and used to expose a sheet of X-ray photographic film for 12 to 24 hours, after which time the X-ray film is developed in the customary manner and examined for the degree of darkening in positions corresponding to the various wells and samples.

Wherever radio-labelled antibody has reacted with antigen (i.e. a positive clinical specimen) a black dot develops on the film, and the density of the dot is dependent upon the amount of antigen trapped and on the time of exposure of the X-ray film to the treated nitrocellulose film. The rest of the X-ray sheet remains unchanged, so positive reactions can be detected easily and quickly by eye.

If the effect on the X-ray film is too faint, the nitrocellulose film can be used to expose another sheet of X-ray film for a longer time so as to obtain a darker dot to assist evaluation.

EXAMPLE 2:

Preparation of clinical sample for test:

Clinical specimens (for example swabs taken of a mucous surface to be assessed, for example from urethra, cervix or conjunctiva) are placed in conventional Chlamydia transport medium (sucrose-phosphate buffer with appropriate antibiotics) and transported to the laboratory. The specimens are numbered, and aliquots are removed for culture if comparative studies are being carried out.

Protease (proteinase K, Sigma P-0390, obtained from Sigma Chemicals) is then added to the remainder of the specimen (final concentration 250 $\mu$g ml$^{-1}$) which is vortexed (conveniently on a "Whirlimixer," for about 10 seconds) and then held at 56 degrees C. for 30 minutes to allow the enzyme to act. The enzyme is then deactivated bu steam treatment in a pressure cooker for 15 minutes.

The prepared specimen is then ready for loading into the Bio-dot plate for the test procedure.

Test procedure:

A 96 well Biodot microfiltration apparatus (Bio-Rad Laboratories Ltd., Watford WDI.8A, United Kingdom) is then assembled with nitrocellulose membrane moistened with phosphate buffered saline (PBS) and specimens are added to the wells (0.4 ml of specimen, 1 specimen per well), then drawn through the membrane by suction. The membrane is removed and placed in blocking solution (PBS which contains 5% W/V skimmed milk powder) for 30 minutes at 37 degrees C., washed in PBS containing 0.05% V/V of "Tween 20" (PBS-T ("Tween 20" is a commercially available surfactant) then incubated with $^{125}$I-labelled genus monoclonal antibody diluted in PBS-T (2-3 x 10$^6$ cpm) for 2.5 hours at 37 degrees C. or at room temperature overnight. The membrane is then thoroughly washed in PBS-T, dried and autoradiographed with pre-flashed Kodak X-Omat AR film and "Super-rapid" screens (exposure time 24 hours at -70 degrees C.). A two-fold titration of a cell culture stock of Chlamydia trachomatis is included as positive control on each membrane. The dilutions are adjusted to yield a range of dots of varying intensity with the end-point at the fifth dilution. Clinical specimens which produce a reaction equal to or greater than the end-point are considered positive.

The monoclonal antibody used was the genus monoclonal antibody J12 used in the commercially available Boots Celltech antigen detection kit ("IDEIA"), which was made and characterized by Dr. Margaret Thornley in Cambridge.

Assessment of the results showed that this procedure was very much more sensitive than the commercial ELISA ("IDEIA"), and also more sensitive than the corresponding test procedure in which the enzyme (protease) treatment was omitted. Sensitivity, specificity, and positive and negative predictive values were all good.

This procedure, using the enzyme treatment, was also appreciably more sensitive than culture methods for diagnosis of ocular Chlamydia trachomatis infection. The reason for this is not clear, but is probably because the procedure overcomes the effect of the topical antibiotics (for example chloramphenicol) which are frequently used to treat patients at the time that swabs are taken.

The names "Tween" and "Whirlimixer" are Registered Trade Marks, and are acknowledged as such.

## Claims

1. Diagnostic method for the diagnosis of infection by an infectious agent with a lipopolysaccharide antigen, in which the sample under examination is first intimately applied to a contacting absorptive medium which has an affinity for a lipopolysaccharide, which then absorbs the lipopolysaccharide antigen of the infectious agent, and the resulting support carrying the absorbed antigen then is treated with a solution containing an antibody which (a) has an affinity for the said antigen and also (b) is associated with means whereby the presence of the antibody (and consequently of any antigen to which it is attached) can be rendered detectable, wherein the clinical sample, before it is applied to the contacting absorptive medium, is treated with a proteolytic enzyme.

2. Diagnostic method as claimed in Claim 1 wherein the infectious agent is one with an epitope on a lipopolysaccharide antigen.

3. Diagnostic method as claimed in Claim 1 or Claim 2 wherein the infectious agent is an organism of the genus Chlamydia.

4. Diagnostic method as claimed in Claim 3 wherein the organism is Chlamydia trachomatis.

5. Diagnostic method as claimed in Claim 3 wherein the organism is Chlamydia psittaci.

6. Diagnostic method as claimed in any one of Claims 1 to 5 wherein the sample for examination is in the form of a dispersion and/or solution in an aqueous medium.

7. Diagnostic method as claimed in in any one of Claims 1 to 6 wherein, after the treatment with the proteolytic enzyme, and before it is applied to the contacting absorptive medium, the specimen is treated in order to deactivate the enzyme.

8. Diagnostic method as claimed in Claim 7 wherein the deactivating treatment is a heating treatment at 100 degrees C or higher.

9. Diagnostic method as claimed in any one of Claims 1 to 8 wherein the intimate contacting of the sample under examination with the contacting absorptive medium is achieved by using the contacting absorptive medium in a form of high surface area.

10. Diagnostic method as claimed in Claim 9 wherein the contacting absorptive medium is in the form of a membrane.

11. Diagnostic method as claimed in any one of Claims 1 to 10 wherein the liquid sample is impelled through a layer of the contacting absorptive medium.

12. Diagnostic method as claimed in Claim 11 wherein the contacting absorptive medium is in the form of a membrane.

13. Diagnostic method as claimed in any one of Claims 1 to 12 wherein the contacting absorptive medium is nitrocellulose.

14. Diagnostic method as claimed in any one of Claims 1 to 13 wherein the antibody used to treat antigen-bearing contacting absorptive material is a monoclonal antibody.

15. Diagnostic method as claimed in any one of Claims 1 to 14 wherein the means associated with the antibody, whereby ' the presence of the antibody (and consequently of any antigen to which it is attached) can be rendered detectable, is radio-labelling.

16. Diagnostic method as claimed in Claim 15 wherein the antibody is labelled with radioactive iodine (Isotope $^{125}$I).

17. Diagnostic method as claimed in any one of Claims 1 to 16 wherein the contacting absorptive medium, after it has absorbed the lipopolysaccharide antigen from the sample, is then treated with a material which blocks substantially all the remaining attachment sites and prevents it trapping any further antigens or materials which can obscure the absorbed lipopolysaccharide antigen.

18. Diagnostic method as claimed in Claim 17 wherein the material which blocks substantially all the remaining attachment sites is a solution of high protein content.

19. Diagnostic test as claimed in any one of Claims 1 to 18 wherein the method is used for an immune dot technique (or immunobinding technique).

## Patentansprüche

1. Diagnostische Methode zur Diagnose einer Infektion durch einen infektiösen Keim mit einem Lipopolysaccharidantigen, in dem die der Prüfung unterzogene Probe zuerst innig auf ein absorptives Kontaktmedium aufgetragen wird, das eine Affinität zu einem Lipopolysaccharid hat; das dann das Lipopolysaccharidantigen des infektiösen Keims absorbiert, und der sich ergebende Träger, der das absorbierte Antigen trägt, wird dann mit einer Lösung behandelt, die einen Antikörper enthält, der (a) eine Affinität zu dem genannten Antigen hat, und außerdem (b) mit einem Medium assoziiert ist, wonach die Anwesenheit des Antikörpers (und folglich jedes Antigens, an das er gebunden ist) nachweisbar gemacht werden kann, wobei die klinische Probe vor ihrer Auftragung auf das absorptive Kontaktmedium mit einem proteolytischen Enzym behandelt wird.

2. Diagnostische Methode, wie unter Anspruch 1 beansprucht, wonach der infektiöse Keim einer mit einem Epitop auf einem Lipopolysaccharidantigen ist.

3. Diagnostische Methode, wie unter Anspruch 1 oder Anspruch 2 beansprucht, wonach der Infektiöse Keim ein Organismus des Genus Chlamydia ist.

4. Diagnostische Methode, wie unter Anspruch 3 beansprucht, wonach der Organismus Chlamydia trachomatis ist.

5. Diagnostische Methode, wie unter Anspruch 3 beansprucht, wonach der Organismus Chlamydia psittaci ist.

6. Diagnostische Methode, wie unter einem der Ansprüche 1 bis 5 beansprucht, wonach die zu untersuchende Probe die Form einer Dispersion bzw. Lösung in einem wäßrigen Medium hat.

7. Diagnostische Methode, wie unter einem der Ansprüche 1 bis 6 beansprucht, wonach die Probe nach Behandlung mit dem protelytischen Enzym und vor der Auftragung auf das absorptive Kontaktmedium behandelt wird, um das Enzym zu desaktivieren.

8. Diagnostische Methode, wie unter Anspruch 7 beansprucht, wonach die desaktivierende Behandlung eine Hitzebehandlung bei 100°C oder höher ist.

9. Diagnostische Methode, wie unter einem der Ansprüche 1 bis 8 beansprucht, wonach die innige Berührung der zu prüfenden Probe mit dem absorptiven Kontaktmedium dadurch erzielt wird, daß das absorptive Kontaktmedium in der Form einer großen Oberfläche verwendet wird.

10. Diagnostische Methode, wie unter Anspruch 9 beansprucht, wonach das absorptive Kontaktmedium eine Membran ist.

11. Diagnostische Methode, wie unter einem der Ansprüche 1 bis 10 beansprucht, wonach die flüssige Probe durch eine Schicht des absorptiven Kontaktmediums gedrückt wird.

12. Diagnostische Methode, wie unter Anspruch 11 beansprucht, wonach das absorptive Kontaktmedium eine Membran ist.

13. Diagnostische Methode, wie unter einem der Ansprüche 1 bis 12 beansprucht, wonach das absorptive Kontaktmedium Nitrozellulose ist.

14. Diagnostische Methode, wie unter einem der Ansprüche 1 bis 13 beansprucht, wonach der zur Behandlung des antigenhaltigen Materials, das das absorptive Material berührt, verwendete Antikörper ein monoklonaler Antikörper ist.

15. Diagnostische Methode, wie unter einem der Ansprüche 1 bis 14 beansprucht, wonach die mit dem Antikörper assoziierten Mittel, wodurch die Anwesenheit des Antikörpers (und folglich jeglichen Antigens, an den es befestigt ist) nachweisbar gemacht werden kann, radioaktive Markierung ist.

16. Diagnostische Methode, wie unter Anspruch 15 beansprucht, wonach der Antikörper mit radioaktivem Jod (Isotop $^{125}$I) markiert ist.

17. Diagnostische Methode, wie unter einem der Ansprüche 1 bis 16 beansprucht, wonach das absorptive Kontaktmedium, nachdem es das Lipopolysaccharidantigen von der Probe absorbiert hat, anschließend mit einem Material behandelt wird, das im wesentlichen alle verbleibenden Befestigungsstellen blockiert und es daran hindert, etwaige weitere Antigene oder Materialien die das absorbierte Lipopolysaccharidantigen verdecken können, einzufangen.

18. Diagnostische Methode, wie unter Anspruch 17 beansprucht, wonach das Material, das im wesentlichen alle verbleibenden Befestigungsstellen blockiert, eine Lösung mit hohem Elweißgehalt ist.

19. Diagnostische Methode, wie unter einem der Ansprüche 1 bis 18 beansprucht, worin die Methode für eine Immunpunkttechnik (oder Immunbindetechnik) verwendet wird.

## Revendications

1. Méthode de diagnostic pour déceler une infection due à un agent infectieux présentant un lipopolysaccharide comme antigène, méthode selon laquelle l'échantillon à examiner est d'abord mis en contact intime avec un milieu de contact absorbant qui a une affinité pour le lipopolysaccharide et qui absorbe alors le lipopolysaccharide antigène de l'agent infectieux, après quoi le support obtenu, qui porte l'antigène absorbé, est traité avec une solution contenant un anticorps qui (a) a une affinité pour ledit antigène et qui (b) est aussi associé à des moyens par lesquels la présence de l'anticorps (et par conséquent de tout antigène auquel il est attaché) peut être rendue détectable, et selon laquelle l'échantillon clinique, avant d'être mis en contact avec le milieu de contact absorbant, est traité avec une enzyme protéolytique.

2. Méthode de diagnostic selon la revendication 1, dans laquelle l'agent infectieux est un agent avec un épitope sur un lipopolysaccharide antigène.

3. Méthode de diagnostic selon la revendication 1 ou 2, dans laquelle l'agent infectieux est un organisme du genre Chlamydia.

4. Méthode de diagnostic selon la revendication 3, dans laquelle l'organisme est la Chlamydia trachomatis.

5. Méthode de diagnostic selon la revendication 3, dans laquelle l'organisme est la <u>Chlamydia</u> <u>psittaci</u>.

6. Méthode de diagnostic selon une des revendications 1 à 5, dans laquelle l'échantillon à examiner est sous forme de dispersion et/ou de solution dans un milieu aqueux.

7. Méthode de diagnostic selon une des revendications 1 à 6, dans laquelle, après le traitement avec l'enzyme protéolytique et avant qu'il soit mis en contact intime avec le milieu de contact absorbant, l'échantillon est traité de manière à désactiver l'enzyme.

8. Méthode de diagnostic selon la revendication 7, dans laquelle le traitement de désactivation est un traitement thermique à 100 degrés C ou davantage.

9. Méthode de diagnotic selon une des revendications 1 à 8, dans laquelle la mise en contact intime de l'échantillon à examiner avec le milieu de contact absorbant est effectuée en mettant en oeuvre ledit milieu de contact absorbant sous une forme présentant une grande surface.

10. Méthode de diagnostic selon la revendication 9, dans laquelle le milieu de contact absorbant est sous forme d'une membrane.

11. Méthode de diagnostic selon une des revendications 1 à 10, dans laquelle l'échantillon liquide est chassé à travers une couche du milieu de contact absorbant.

12. Méthode de diagnostic selon la revendication 11, dans laquelle le milieu de contact absorbant est sous forme d'une membrane.

13. Méthode de diagnostic selon une des revendications 1 à 12, dans laquelle le milieu de contact absorbant est de la nitrocellulose.

14. Méthode de diagnostic selon une des revendications 1 à 13, dans laquelle l'anticorps utilisé pour traiter le matériau absorbant qui porte l'antigène est un anticorps monoclonal.

15. Méthode de diagnostic selon une des revendications 1 à 14, dans laquelle les moyens associés à l'anticorps, grâce auxquels la présence de l'anticorps (et par conséquent de tout antigène auquel il est attaché) peut être rendue détectable, est un marqueur radioactif.

16. Méthode selon la revendication 15, dans laquelle l'anticorps est marqué au moyen d'iode radioactif (isotope $^{125}$I).

17. Méthode de diagnostic selon une des revendications 1 à 16, dans laquelle le milieu de contact absorbant, après qu'il a absorbé le lipopolysaccharide antigène de l'échantillon, est alors traité avec un matériau qui bloque sensiblement tous les sites de fixation qui restent et empêche ainsi que s'y attache tout autre antigène ou matériau qui pourrait occulter le lipopolysaccharide antigène qu'il a absorbé.

18. Méthode de diagnostic selon la revendication 17, dans laquelle le matériau qui bloque sensiblement tous les sites de fixation qui restent est une solution à haute teneur en protéine.

19. Test de diagnostic selon une des revendications 1 à 18, dans lequel la méthode est utilisée en vue d'une technique dite "immune dot" (ou technique d'immunofixation).